(19) Europäisches Patentamt
European Patent Office
Office européen des brevets

(11) **EP 4 163 921 A1**

(12) **EUROPEAN PATENT APPLICATION**

(43) Date of publication:
**12.04.2023   Bulletin 2023/15**

(21) Application number: **21201106.8**

(22) Date of filing: **06.10.2021**

(51) International Patent Classification (IPC):
**G16C 60/00** (2019.01)

(52) Cooperative Patent Classification (CPC):
**G16C 60/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Covestro Deutschland AG**
**51373 Leverkusen (DE)**

(72) Inventor: **Miyazaki, Yu**
**Amagasaki, 661-0977 (JP)**

(74) Representative: **Levpat**
**c/o Covestro AG**
**Gebäude K12**
**51373 Leverkusen (DE)**

(54)   **PROGRAM, INFORMATION PROCESSING APPARATUS, SYSTEM, SERVER, TERMINAL, AND METHOD FOR OBTAINING A RECIPE FOR OBTAINING A TARGET POLYURETHANE FOAM PHYSICAL PROPERTY**

(57)   A program includes a unit to obtain recipe initial information, environmental condition information, and target physical property information, a unit to store a regression formula that defines a relationship between recipe information and environmental condition information, and physical property information, and a unit to derive recipe information produced by applying an error function such that a difference between the target physical property information and expected physical property information is reduced. The expected physical property information is obtained by applying the recipe initial information and the environmental condition information to the regression formula. Each of the physical property information and the target physical property information includes a density and a compressive strength, and the regression formula includes a regression formula for the density and a regression formula for the compressive strength.

Figure 2

Processed by Luminess, 75001 PARIS (FR)

**Description**

**BACKGROUND**

Technical Field

The present invention is a program, an information processing apparatus, a system, a server, a terminal, and/or a method related to information processing on a raw material of a chemical product.

Related Art

In preparation of raw materials for chemical products, it is necessary to generate a recipe (information on raw materials for chemical products) in order to obtain target characteristics.

EP 1158450 A describes a method of creating a recipe for obtaining target characteristics by a computer. However, the method is based on the premise that a relationship between the target characteristics and a mixed weight when various raw materials are used is clear, and a method of creating the recipe is to be created when the premise is different is not described.

In addition, WO 2019/243115 A describes a system for deriving recipe information for obtaining target physical properties of a polymer composition. However, in a reference method, it is necessary for a user to search for a combination of recipe values such that a calculation result by an expectation expression satisfies a target value of the physical properties by trial and error after an equation for expecting physical properties from recipe information is designed.

**SUMMARY**

Even in various cases where the relationship between the target characteristics and the mixed weight when various raw materials are used is not clear, it is preferable to obtain recipes (ratio of NCO to OH and amount of water added) for obtaining target foam physical properties (density and compressive strength). In addition, since it is burdensome for the user to search for the recipe values by trial and error such that the calculation result by the expression satisfies the target value of the physical properties, it is preferable that there are few steps by trial and error.

Thus, the technology according to the present application relates to a program, an information processing apparatus, a system, a server, a terminal, and/or a method related to information processing for obtaining a recipe for obtaining a target foam physical property by a computational approach.

A computer program according to an embodiment of the present application may be a program for causing a computer to function as means for obtaining initial recipe information, environmental condition information, and target physical property information, means for storing a regression formula that defines a relationship between recipe information and environmental condition information, and physical property information, and means for deriving recipe information produced by applying an error function such that a difference between the target physical property information and expected physical property information is reduced. The expected physical property information is obtained by applying the initial recipe information and the environmental condition information to the regression formula.

A method according to another embodiment of the present application may be a method executed by a computer including obtaining initial recipe information, environmental condition information, and target physical property information, storing a regression formula that defines a relationship between recipe information, environmental condition information, and physical property information, and deriving recipe information produced by applying an error function such that a difference between the target physical property information and expected physical property information is reduced. The expected physical property information is obtained by applying the initial recipe information and the environmental condition information to the regression formula.

An apparatus according to still another embodiment of the present application may be an apparatus including a unit to obtain initial recipe information, environmental condition information, and target physical property information, a unit to store a regression formula that defines a relationship between recipe information, environmental condition information, and physical property information, and a unit to derive recipe information produced by applying an error function such that a difference between the target physical property information and expected physical property information is reduced. The expected physical property information is obtained by applying the initial recipe information and the environmental condition information to the regression formula.

According to the embodiment of the present invention, the recipe value can be calculated more appropriately.

**BRIEF DESCRIPTION OF DRAWINGS**

FIG. 1 is a configuration example of an information processing apparatus according to the present application;
FIG. 2 is an example schematically illustrating an outline according to the present application;
FIG. 3 is an example schematically illustrating the outline according to the present application;
FIG. 4 is an example of an input screen of a system according to the present application;
FIG. 5 is an example of a display screen of the system according to the present application;
FIG. 6 is a diagram illustrating a flow of processing of an example of a system according to an embodiment;
FIG. 7 is an example illustrating an attention point according to the present application;
FIG. 8 is a diagram illustrating a flow of processing of an example of the system according to an embodiment;
FIG. 9 is a diagram illustrating a data format of an example of the system according to an embodiment; and
FIG. 10 is a diagram illustrating a data format of an example of the system according to an embodiment.

**DETAILED DESCRIPTION**

1. Function of Information Processing Apparatus

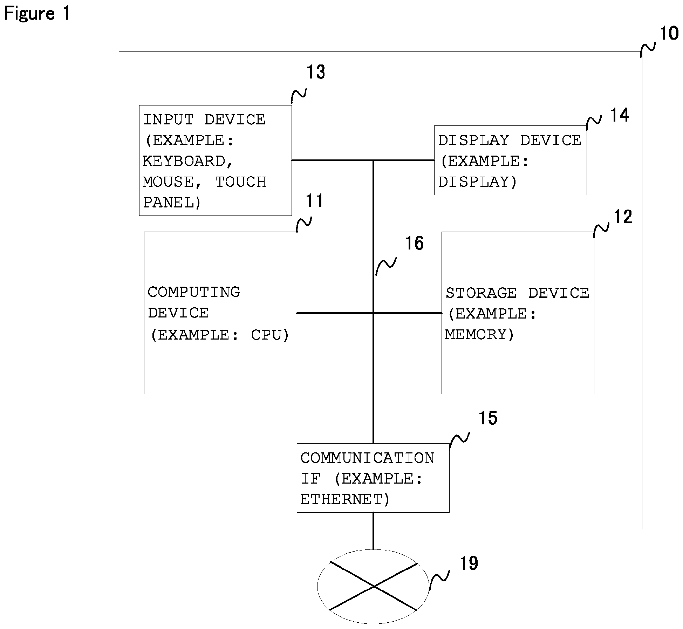

An information processing apparatus according to the present application may have various aspects. An information processing apparatus as an example will be described with reference to FIG. 1. An information processing apparatus 10 may include a computing device 11, a storage device 12, an input device 13, an output device 14, a communication IF 15, and a line 16 connecting these devices.

The computing device is a device capable of executing a command of a program. The computing device may be, for example, a processor, a CPU, an MPU, or the like. The computing device may be capable of sequential computation or parallel computation. The computing device may include a graphics processing unit, a digital signal processor, and the like.

The storage device is a device that records information. The storage may be temporary storage or long-term storage. The storage device may be either an internal memory or an external memory, or both the internal memory and the external memory. The storage device 12 may be a magnetic disk (hard disk), an optical disk, a magnetic tape, a semiconductor memory, or the like. Although not illustrated, the storage device may be a storage device via a network or a storage device on a cloud via a network. The storage device can include a program for executing a technology according to the present application, and can also appropriately record data necessary for executing processing of the technology according to the present application. The storage device may include a database.

The line may be any line as long as information can be transmitted between devices. The line may be, for example, a bus. The bus has a function of transmitting information between the computing device, the storage device, the input device, a display device, and the communication IF.

The input device inputs information. Examples of the input device include an input device such as a keyboard, a mouse, a touch panel, or a pen-type instruction device. Note that, the input device may have other functions such that a touch panel has a display function.

The display device has a function of displaying information. Examples of the display device include a liquid crystal display, a plasma display, an organic EL display, and the like, but in short, any device capable of displaying information may be used. An input device such as a touch panel may be partially provided.

The communication IF has a function capable of communicating information to an external network. The communication IF may have any connection form. For example, USB, IEEE 1394, Ethernet (registered trademark), PCI, SCSI, or the like may be used. Note that, the network may be either a wired network or a wireless network, and in the case of the wired network, an optical fiber, a coaxial cable, or the like may be used.

In the above description, the case where the computing device is executed based on the program provided in the storage device has been described as an example. However, as one of forms in which the bus, the computing device, and the storage device are combined, information processing according to a system of the present application may be realized by a programmable logic device capable of changing a hardware circuit itself or a dedicated circuit in which information processing to be executed is determined. When the information processing is realized by the programmable logic device or the dedicated circuit, there is an advantage that the technology according to the present application can be processed at a higher speed.

The information processing apparatus may be a dedicated information processing apparatus for a part or the whole of the technology according to the present application, or may be an information processing apparatus capable of executing a technology other than the technology according to the present application. The information processing apparatus may be a server, a workstation, a desktop personal computer, a laptop personal computer, a notebook personal computer, a PDA, a mobile phone, a smartphone, or the like.

Although one information processing apparatus has been described in FIG. 1, the information processing apparatus capable of processing information according to the present application may include a plurality of information processing apparatuses. The plurality of information processing apparatuses may be internally connected or may be externally connected via a network. When the information processing apparatus includes the plurality of information processing apparatuses, even though owners or administrators are different, the information processing apparatuses may be in a state of being available due to having an access right or the like when the technology described in the present processing is executed. The information processing apparatus may be a physical entity or a virtual entity. For example, the information processing apparatus may be virtually realized by using cloud computing. FIG. 1 illustrates a basic configuration of the information processing apparatus according to the present application. However, when the information processing apparatus cooperates with another information processing apparatus, the information processing apparatus may not include the input device or the display device. Note that, in the present application, the term system may be used for a system including one or a plurality of information processing apparatuses. The term system may include a part or the whole of the technology according to the present application. The term system may indicate only a server, only a terminal, a server and a terminal, only a cloud, or a cloud and a terminal.

2. Outline of Technology according to Present Application

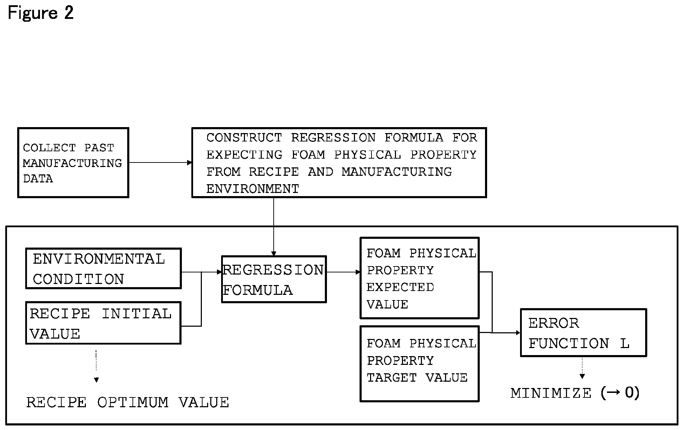

FIG. 2 schematically illustrates an outline of the technology according to the present application. There is a case where past manufacturing data is collected and a regression formula for expecting a physical property of a foam can be constructed from a recipe and a manufacturing environmental condition. In this case, initial values of the recipe and the environmental condition are substituted into the regression formula, and thus, the physical property of the foam when such a recipe is used can be expected. Here, when a target value of the physical property of the foam is given, there is a case where an optimum value of the recipe corresponding to the foam physical property target value can be derived by applying an error function L such that an error between a foam physical property expected value and the foam physical property target value can be minimized. An example embodying such an outline will be described below as a first embodiment.

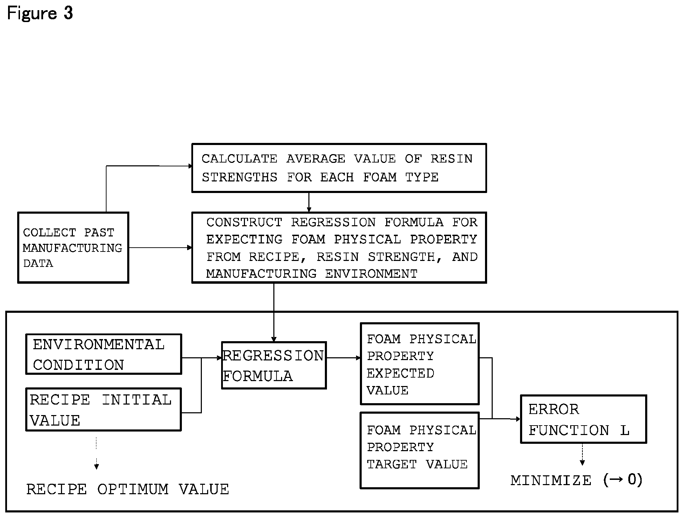

FIG. 3 schematically illustrates an outline of another technology according to the present application. There is a case where past manufacturing data is collected and an average value of resin strengths can be calculated for each foam type from the collected past manufacturing data. There is a case where a regression formula for expecting the physical property of the foam is constructed from the resin strength, the recipe, and the manufacturing environmental condition. As will be described later, the average value of the resin strengths is calculated, and thus, there is an advantage that a regression formula available for various foam types can be constructed. The derivation of the recipe optimum value using such a regression formula is similar to the outline of the above-described first embodiment, and details thereof will be described below as a second embodiment.

3. Embodiments

3.1. Fist Embodiment

Next, a system according to the first embodiment will be described as an embodiment of a system according to the present invention.

The system according to the present embodiment may be a program including a plurality of functions including macros executed on Excel. In this case, the program including the functions has an advantage that functions implemented in Excel can be used.

First, the system according to the present embodiment obtains a foam type, target physical property information, environmental condition information, and recipe initial information. Note that, in the present document, information to be substituted into the regression formula, such as the environmental condition information and the recipe initial information, may be referred to as "regression formula input information". The system according to the present embodiment implemented in the macros on Excel may obtain these pieces of information by using an input function of Excel.

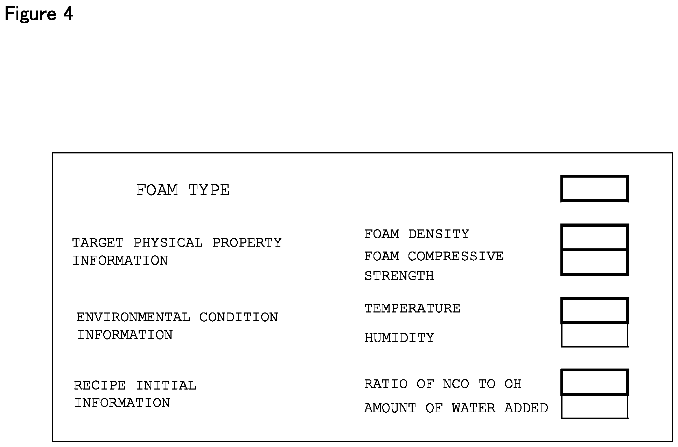

FIG. 4 is a diagram illustrating an example in which input information is received in the system according to the present embodiment. In the present example, the system according to the present embodiment is configured to be able to receive inputs of the target physical property information, the environmental condition information, and the recipe initial information.

The foam type is information for specifying a foam desired to be manufactured by a user of the present system.

The target physical property information is information on characteristics of the foam desired to be manufactured by the user of the present system. The target physical property information may include a density of the foam and a compressive strength of the foam.

Here, the foam may be a polyurethane foam. The polyurethane foam may be a flexible or rigid foam. In the present specification, the term "flexible polyurethane foam" refers to a urethane foam molded by using a blended polyol containing 50 mass% or more of a polyol having a hydroxyl value of 56 mg KOH/g or less and having a product density of less than 100 kg/m$^3$. The "rigid polyurethane foam" refers to a urethane foam molded by using a blended polyol containing 50 mass% or more of a polyol having a hydroxyl value of 300 to 500 mg KOH/g.

The foam according to the present application may be variously used, and the use thereof is not limited. For example, the technology according to the present application may be a foam for furniture, a vehicle material, or a consumer product (sponge, cushion, or the like) as the flexible foam, and may be a foam for a building material, a vehicle material, or the like as the rigid foam.

The recipe initial information may be information initially required in a manufacturing apparatus that manufactures the foam. For example, the recipe initial information may include a ratio of NCO to OH and/or an amount of water added. The ratio of NCO to OH may be a ratio in a predetermined unit. The amount of water added may be an amount of water added per predetermined unit, or may be information expressed by weight or volume. The recipe initial information may be set based on past knowledge and experience of the user of the present system, or may be a value temporarily input even when the user of the present system does not have past knowledge and experience. The recipe initial information may be the recipe initial information or the target physical property information used when the present system is previously used, or may be information obtained from another system by cooperating with another system. The recipe initial information may be a set of a ratio of NCO to OH and an amount of water added corresponding to a portion selected by the user in an area where a physical property error to be described later is displayed.

The environmental condition information may include a temperature, a pressure, and/or a humidity.

The target physical property information may include the density of the foam and/or the compressive strength of the foam.

Subsequently, the system according to the present embodiment substitutes the obtained regression formula input information into the regression formula. The regression formula may be, for example, the following equations. Here, Equations 1 to 3 are illustrated as the regression formula, but the regression formula to be used is not limited thereto, and may be in various aspects. Note that, in these equations, X1 represents an index (ratio of NCO to OH), X2 represents an amount of water, X3 represents an absolute humidity, X4 represents a pressure, X5 represents an air temperature, Y1 represents a foam density, Y2 represents a compressive strength, and w and v represent coefficients. The coefficients of w and v may be determined manually by a person in advance using information related to the foam or may be determined by an automatic calculation method, and the coefficients of w and v may be determined by using, for example, sparse regression in addition to simple linear regression, or may be determined by using Lasso or Elastic Net.

[Math. 1]

$$Y1 = w0 + w1*X1 + w2*X2 + w11*X12 + w22*X22 + w12*X1*X2 + w3*X3 + w4*X4 + w5*X5$$
$$Y2 = v0 + v1*X1 + v2*X2 + v11*X12 + v22*X22 + v12*X1*X2 + v3*X3 + v4*X4 + v5*X5$$

[Math. 2]

$$Y1 = w0 + w1*X1 + w2*X2 + w11*X12 + w22*X22 + w12*X1*X2 + w3*X3 + w4*X4 + w5*X5 + wb*Xb + wc*Xc$$
$$Y2 = v0 + v1*X1 + v2*X2 + v11*X12 + v22*X22 + v12*X1*X2 + v3*X3 + v4*X4 + v5*X5 + vb*Xb + vc*Xc$$

[Math. 3]

$$Y1 = w0 + w1*X1 + w2*X2 + w11*X12 + w22*X22 + w12*X1*X2 + w3*X3 + w4*X4 + w5*X5 + wb*Xb + w1b*X1*Xb + w2b*X2*Xb + w3b*X3*Xb + w4b*X4*Xb + w5b*X5*Xb + wc*Xc + w1c*X1*Xc + w2c*X2*Xc + w3c*X3*Xc + w4c*X4*Xc + w5c*X5*Xc$$
$$Y2 = v0 + v1*X1 + v2*X2 + v11*X12 + v22*X22 + v12*X1*X2 + v3*X3 + v4*X4 + v5*X5 + vb*Xb + v1b*X1*Xb + v2b*X2*Xb + v3b*X3*Xb + v4b*X4*Xb + v5b*X5*Xb + vc*Xc + v1c*X1*Xc + v2c*X2*Xc + v3c*X3*Xc + v4c*X4*Xc + v5c*X5*Xc$$

The above-described illustrated regression formulas may be able to express the density and the compressive strength for the foam. These regression formulas may be constructed such that a person can accurately express a specific foam based on information regarding a specific foam type (the environmental conditions such as a humidity, an atmospheric pressure, and a temperature, the recipe initial values such as the amount of water added and the ratio of NCO to OH, and the physical property information such as the density and the compressive strength of the foam), and may be constructed so as to set coefficients in advance such that the person can express the specific foam. The system according to the present embodiment may store the regression formula with the coefficients set in this manner and the corresponding foam type in association with each other. The system according to the present embodiment may use the regression formula corresponding to the above-described input foam type.

Subsequently, the system according to the present embodiment calculates the recipe information that reduces a difference between the density of the foam and the compressive strength of the foam based on the recipe initial information and a density and a compressive strength in the target physical property information (in the present document, may be referred to as a "physical property error") by using the density of the foam and the compressive strength of the foam based on the recipe initial information, the density and the compressive strength in the target physical property information, and the error function, which are obtained by the above-described substitution.

Here, various methods for calculating an error between two arguments may be used as the error function for calculating the physical property error. Here, since an error between a plurality of values such as two arguments is calculated, when an attempt is made to simply eliminate the error by subtraction, a value having a large absolute value is emphasized. Thus, the plurality of values such as two arguments is normalized and then the error is calculated. Accordingly, there is an advantage that the error between the plurality of values can be appropriately evaluated instead of the error function that simply emphasizes the value having the large absolute value.

The system according to the present embodiment may use a generalized reduced gradient method for the regression formula implemented in Excel.

The system according to the present embodiment derives the recipe information (ratio of NCO to OH and amount of water) corresponding to the target physical property information (density and compressive strength) that reduces the physical property error, and the recipe information is generated by using the generalized reduced gradient method, the regression formula, and the error function. Note that, here, a case where the recipe information is "derived" is a term of a superordinate concept including both a case where the recipe information (ratio of NCO to OH and amount of water) is obtained based on the generalized reduced gradient method implemented in the system according to the present embodiment and a case where the recipe information is obtained based on the generalized reduced gradient method implemented outside the system according to the present embodiment. The recipe information (ratio of NCO to OH and amount of water) corresponding to the target physical property information (density and compressive strength) in which the physical property error is reduced may be recipe information corresponding to target physical property information in which the physical property error is reduced to a predetermined value or less, or may be recipe information corresponding to target physical property information in which the physical property error is reduced to zero.

As described above, in a manufacturing line of the form, there are the recipes (ratio of NCO to OH and amount of water added) for obtaining the physical property of the target foam (density and compressive strength). However, since the foam physical property is also affected by surrounding environments (temperature, humidity, and the like) of the manufacturing line, the foam physical property obtained even when the foam is manufactured according to the recipe is often deviated from the target value. Although it can be expected that the deviation is resolved somewhat by the knowledge and experience of a manufacturer, a non-personal recipe optimization method by a computational approach has been desired in order to further stabilize the quality. Since the technology according to the present application is a computational approach using the above-described regression formula and generalized reduced gradient method, there is an advantage that the recipe information (ratio of NCO to OH and amount of water) corresponding to the target physical property information (density and compressive strength) in which the physical property error is reduced can be derived in a non-individual manner as described above. In addition, when the physical property information can be expressed with a certain degree of accuracy by the regression formula, the present invention can be applied even when a relationship between the target characteristics and a mixed weight when various raw materials are used is not clear, and since the user does not need to search by trial and error, there is an advantage that the recipe can be created without a burden on the user.

Note that, the system according to the present embodiment may have a configuration for displaying the physical property error corresponding to the recipe initial value.

For example, the system according to the present embodiment may have a configuration for displaying Cartesian coordinates in which a vertical axis represents the ratio of NCO to OH and a horizontal axis represents the amount of water added. Here, in such Cartesian coordinates, values corresponding to each ratio of NCO to OH and each amount of water added may be physical property errors between the physical property information (density and compressive strength) when the ratio of NCO to OH and the amount of water added are applied to the regression formula and the input target physical property information.

Such physical property errors may be distinguished and displayed in various aspects. For example, information for distinguishing and displaying the physical property errors may include at least one of a color, a pattern, and the like as a value corresponding to each ratio of NCO to OH and each amount of water added in the Cartesian coordinates. In this case, the information for distinguishing and displaying the physical property errors may be displayed so as to correspond to a physical property error in a predetermined range. For example, when the information is the color, the color is color 1 when the physical property error is less than 0.5, and the color is color 2 when the physical property error is 0.5 or more and less than 1.0. In addition, a display for describing that these pieces of information indicate predetermined physical property errors may be displayed. For example, as the above-described example, information such as color 1 when the physical property error is less than 0.5 and color 2 when the physical property error is 0.5 or more and less than 1.0 may be displayed.

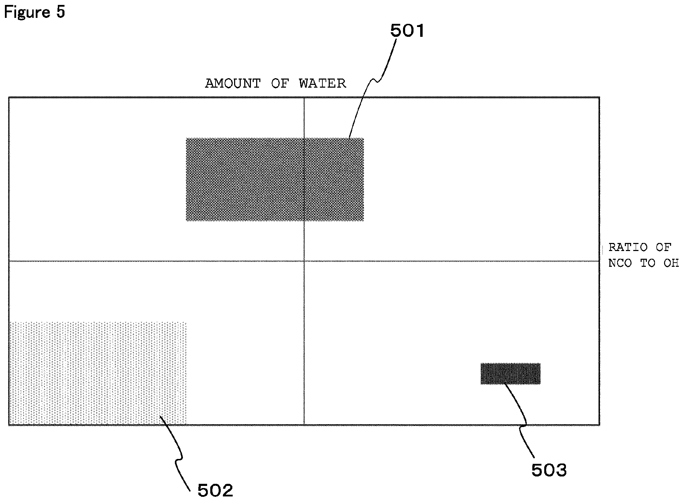

FIG. 5 illustrates an aspect of displaying such physical property errors. In this drawing, in the Cartesian coordinates, regions 501 to 503 are displayed in aspects different from the other regions, and differences between the physical property errors are displayed. In this example, in particular, the region 503 is displayed in a different aspect by a darker display than the other regions 501 and 502, and is drawn as an example indicating that the physical property error is small.

When the system according to the present embodiment includes a configuration for displaying the physical property error corresponding to the recipe initial value, the following processing may be performed.

First, the system according to the present embodiment obtains a foam type, target physical property information, environmental condition information, and recipe initial information.

Subsequently, the system according to the present embodiment specifies the regression formula corresponding to the foam type.

Subsequently, the system according to the present embodiment sets a range to which the recipe information can be applied based on the recipe initial information. For example, in the system according to the present embodiment, values obtained by increasing or decreasing each ratio of NCO to OH and each amount of water added based on the recipe initial value by predetermined amounts are set as lower limit values and upper limit values of the ratio of NCO to OH and the amount of water added. Note that, these values may correspond to lower limit values and upper limit values of the Cartesian coordinates in the display indicating the physical property error.

The system according to the present embodiment generates the corresponding physical property information by using the environmental condition information and the regression formula corresponding to the above-described foam type for each combination of values based on predetermined intervals within the ranges of the lower limit values and the upper limit values of the above-described ratio of NCO to OH and amount of water added.

For example, a case where the lower limit value and the upper limit value of the ratio of NCO to OH are set to 80 to 150, the lower limit value and the upper limit value of the amount of water added are set to 300 to 500, the predetermined interval of the ratio of NCO to OH is 10, and the predetermined interval of the water addition amount is 30 is considered. In this case, the system according to the present embodiment generates the physical property information by using the environmental condition information, the regression formula corresponding to above-described the foam type, the ratio of NCO to OH of 80, and the amount of water added of 300, and sets the physical property information as physical property information of a portion associated with the ratio of NCO to OH of 80 and the amount of water added of 300. Subsequently, the system according to the present embodiment generates the physical property information by using the environmental condition information, the regression formula corresponding to the above-described foam type, the ratio of NCO to OH of 90, and the amount of water added of 330, and sets the physical property information as physical property information of a portion associated with the ratio of NCO to OH of 90 and the water addition amount of 330. Thus, the physical property information is generated at predetermined intervals within the ranges of the ratio of NCO to OH and the water addition amount. Note that, here, the predetermined amounts for setting the lower limit values and the upper limit values of the ratio of NCO to OH and the amount of water added and/or the predetermined intervals for calculating the physical property information may be stored in advance in the system according to the present embodiment, or may be values input by the user.

Subsequently, the system according to the present embodiment may display the physical property errors by using physical property errors that are a difference between each physical property information and the target physical property information as values of portions of the corresponding ratio of NCO to OH and amount of water added.

In such a case, there is an advantage that the user can understand at a glance the physical property error when the recipe initial values input by the user is input without inputting other recipe initial values other than the recipe initial values input by the user. In particular, there is an advantage that the user can notice that the recipe initial value in which the physical property error cannot be reduced is selected in some regression formulas to be applied and some generalized reduced gradient methods to be applied.

Note that, although the Cartesian coordinates have been described as the configuration for displaying the physical property error, a graph including a coordinate system capable of displaying three components capable of displaying values corresponding to each ratio of NCO to OH and each amount of water added may be used, and the configuration is not limited to the Cartesian coordinates. For example, the coordinates may be cylindrical coordinates, spherical coordinates, elliptical coordinates, parabolic coordinates, or the like, or these coordinates may be modified.

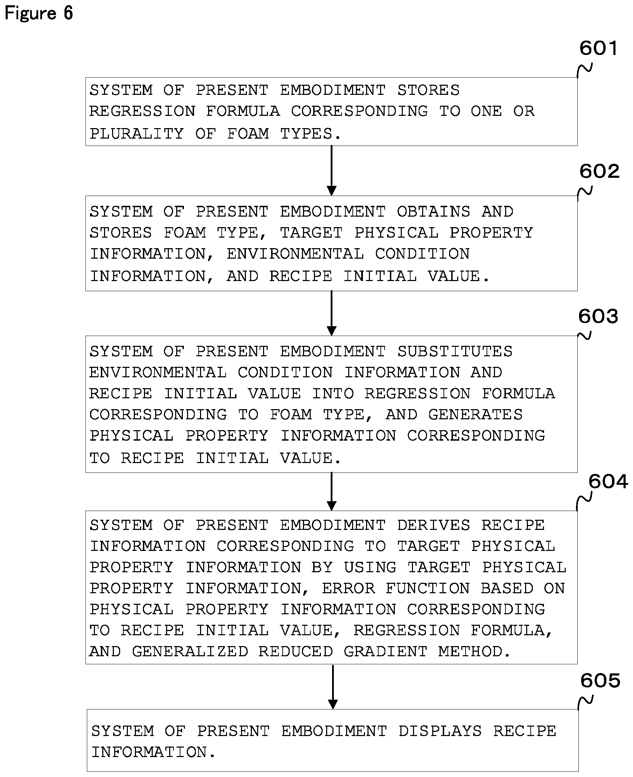

The flowchart of FIG. 6 briefly illustrates a flow of the system according to the present embodiment.

Although the example in which the system according to the present embodiment is implemented by the functions executed on Excel has been described above, the system according to the present embodiment is not limited to such an example and may be executed on various systems. In particular, Lasso and Elastic Net may be used as a sparse regression method, and the generalized reduced gradient method may be used as a method of obtaining the recipe optimum value. A system executed on R that is a program execution environment for statistical analysis or Python that is an execution environment may be used.

In addition, the system according to the present embodiment is not limited thereto, and may be an aspect that adopts the generalized reduced gradient method as an implementation form. In this case, there is an advantage that it is not necessary to use the generalized reduced gradient method in the execution environment.

3.2. Second Embodiment

Next, a system according to a second embodiment will be described as the embodiment of the system according to the present invention. The system according to the present embodiment uses one type of regression formula available for various foam types for each physical property information. Note that, only portions different from those of the first embodiment will be described, and description of portions similar to those of the first embodiment will be omitted.

That is, the regression formula corresponding to the foam type is used as the regression formula used in the system according to the first embodiment. In this case, the following problem may occur particularly when the number of foam types used increases. That is, first, when information depending on the foam type is not included in the regression formula, a regression formula suitable for each foam type is different. Thus, when the number of foam types increases, the number of regression formulas increases, implementation becomes difficult, and a burden determined by a person in advance also increases. In addition, it is difficult to construct a regression formula with high expectation possibility for a foam type with few samples. In particular, when an expectation expression incorporating a foam type as a fixed effect is created, the number of explanatory variables becomes enormous as the number of handled foam types increases, and optimal modeling becomes difficult. Thus, there is a problem that the regression formula cannot correspond to an unknown foam type.

Thus, the system according to the second embodiment uses a predetermined number of regression formulas in advance modified to be available for various foam types of the predetermined number or more.

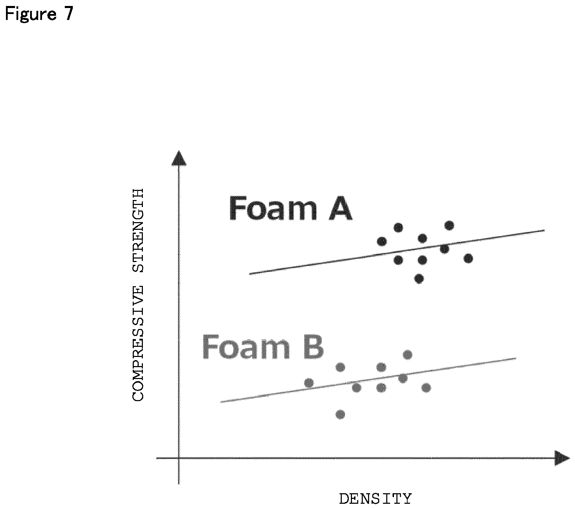

In this regard, the inventors have examined as follows. That is, the compressive strength of the foam is directly proportional to the foam density, but even though the foam density is equal, the compressive strength of the foam increases as the strength of the resin itself is high. For example, FIG. 7 schematically illustrates a relationship between the density of the foam and the compressive strength of the foam. Thus, it can be considered that the compressive strength per density (compressive strength/density) reflects the strength derived from the resin.

From the above viewpoint, the inventors have found that, for example, when one regression formula includes the compressive strength per density (compressive strength divided by density), the compressive strength per density can be changed and used according to the foam type.

When the above-described compressive strength per density is $\sigma\_i$ based on the above technical idea, the following regression formula can be generated.

[Math. 4]

$$Y1 = w0 + w1*X1 + w2*X2 + w11*X12 + w22*X22 + w12*X1*X2 + w3*X3 + w4*X4 + w5*X5 + ws*\sigma\_i$$

$$Y2 = v0 + v1*X1 + v2*X2 + v11*X12 + v22*X22 + v12*X1*X2 + v3*X3 + v4*X4 + v5*X5 + vs*\sigma\_i$$

In Equation 4, the regression formula can correspond to the foam type by $ws*\sigma\_i$ and $vs*\sigma\_i$.

[Math. 5]

$$Y1 = w0 + w1*X1 + w2*X2 + w11*X12 + w22*X22 + w12*X1*X2 + w3*X3 + w4*X4 + w5*X5 + ws*\sigma\_i + w1s*X1*\sigma\_i + w2s*X2*\sigma\_i + w3s*X3*\sigma\_i + w4s*X4*\sigma\_i + w5s*X5*\sigma\_i$$

$$Y2 = v0 + v1*X1 + v2*X2 + v11*X12 + v22*X22 + v12*X1*X2 + v3*X3 + v4*X4 + v5*X5 + vs*\sigma\_i + v1s*X1*\sigma\_i + v2s*X2*\sigma\_i + v3s*X3*\sigma\_i + v4s*X4*\sigma\_i + v5s*X5*\sigma\_i$$

Here, the regression formula corresponding to the foam type can be used by $ws*\sigma\_i + w1s*X1*\sigma\_i + w2s*X2*\sigma\_i + w3s*X3*\sigma\_i + w4s*X4*\sigma\_i + w5s*X5*\sigma\_i$ in Y1, and $vs*\sigma\_i + v1s*X1*\sigma\_i + v2s*X2*\sigma\_i + v3s*X3*\sigma\_i + v4s*X4*\sigma\_i + v5s*X5*\sigma\_i$ in Y2. Equation 5 can also be expressed when there is an interaction between the compressive strength per density and the recipe information and/or the environmental condition information.

Note that, the selection of Equations 4 and 5 and the coefficients w and v of these equations may be selected based on values determined in advance by a person or may be selected by automatic processing. For example, a model without the interaction of Equation 4 and a model with the interaction of Equation 5 may regress learning data in each model, learning data with a higher determination coefficient or learning data with an expected error average may be adopted, and the learning data may be selected by automatic processing.

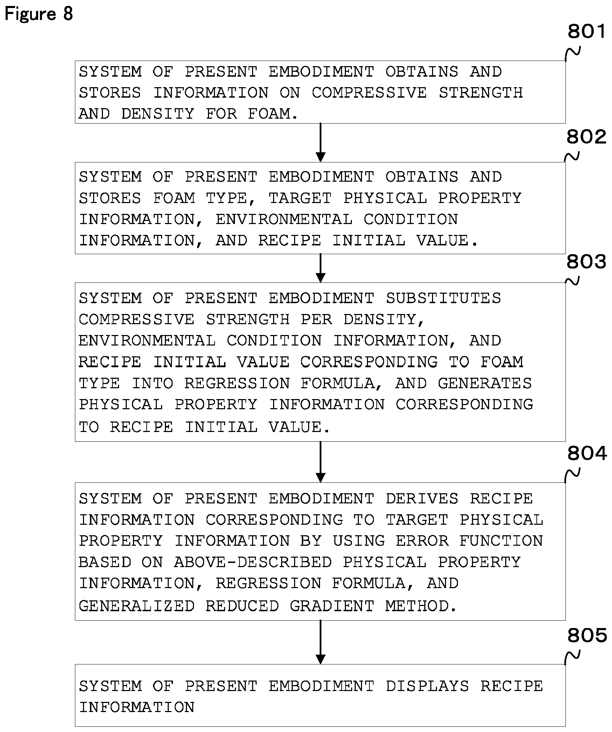

Next, a flow of processing of the system according to the present embodiment using the compressive strength per density described above will be described with reference to FIG. 8.

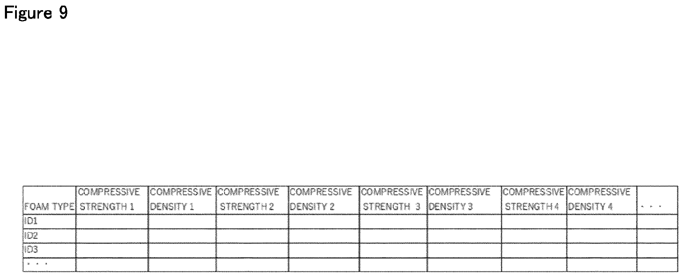

First, the system according to the present embodiment obtains and stores information on the compressive strength and the density for the foam. Here, the information on the compressive strength and the density of the foam may be obtained by a file or the like including information on the foam. For example, FIG. 9 illustrates an example in which a plurality of sets of compressive strengths and densities (compressive strength 1 and density 1, compressive strength 2 and density 2, and the like) is associated with the foam types (foam types ID1 to ID3). The system according to the present embodiment may calculate the compressive strength per density corresponding to one foam type based on such a file. In this case, when there is the plurality of sets of compressive strengths and densities, the compressive strength per density may be calculated by calculating an average value thereof. In addition, a regression formula for Equations 4 and 5 described above may be automatically generated by using the information on the compressive strength and the density. Here, as described above, for example, in the regression formula, the coefficients of w and v may be dynamically determined by an automatically calculated method by using the information on the compressive strength and the density, and the coefficients of w and v may be determined by using, for example, sparse regression in addition to simple linear regression, and may be determined by using Lasso or Elastic Net which is the related art. Note that, as described above, the regression formula manually or automatically constructed in advance may be used as these regression formulas instead of the automatic and dynamic calculation. When the regression formula is prepared in advance, the regression formula corresponding to the target foam may be specified by using the above-described compressive strength per density. For example, the regression formula corresponding to each of the plurality of foams may be manually or automatically constructed in advance, and a regression formula related to the compressive strength per density corresponding to the compressive strength per density based on the obtained information on the compressive strength and the density may be specified among the compressive strengths per density in these regression formulas. Here, as the correspondence of the compressive strength per density, the compressive strength per density closer to the obtained compressive strength per density than other compressive strengths per density may be selected from among the compressive strengths per density in each regression formula. Here, the closeness may be closeness with a small difference between the compressive strengths per density.

In addition, when the information on the compressive strength and the density of such a foam is not obtained, the system according to the present embodiment may calculate the compressive strength per density based on target physical property information (that is, a compressive strength and a density of a target foam) to be input next. In this case, the regression formula may be calculated or specified as in the information on the compressive strength and the density based on the file described above.

Subsequently, the system according to the present embodiment obtains and stores the foam type, the target physical property information, the environmental condition information, and the recipe initial value. Here, the foam type may correspond to the foam type in the information on the compressive strength and the density for the above-described foam. Note that, when the information on the compressive strength and the density of the foam is information of only one foam type or when the compressive strength per density is calculated from the target physical property information, the information of the foam type may not be obtained.

Subsequently, the system according to the present embodiment substitutes the compressive strength per density, the environmental condition information, and the recipe initial value corresponding to the foam type into the regression formula, and generates the physical property information corresponding to the recipe initial value. Here, as the regression formula, Equation 4 or Equation 5 may be selected by automatic processing.

Hereinafter, similarly to the first embodiment, the system according to the present embodiment derives the recipe information corresponding to the target physical property information by using the error function based on the above-described physical property information, the regression formula, and the generalized reduced gradient method, and displays the recipe information. In addition, similarly to the first embodiment, the physical property errors corresponding to a plurality of different ratios of NCO to OH and amounts of water added may be calculated, and these physical property errors may be displayed.

3.3. Third Embodiment

A system according to a third embodiment is a case where the system according to the first or second embodiment is performed in a server-client manner.

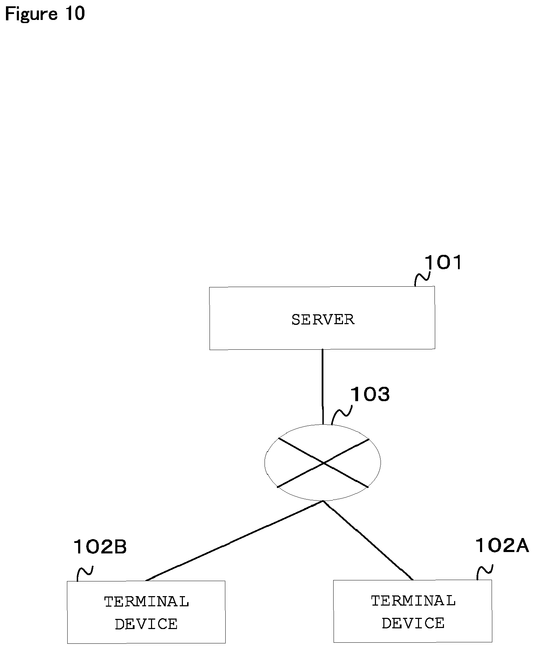

Referring to FIG. 10, in the system according to the third embodiment, a server 101 and terminal devices 102A and 102B may be connectable to each other via a network 103. Although a case where there are two terminal devices is illustrated, the number of terminal devices is not limited, and may be one or three or more.

The terminal devices 102A and 102B may mainly perform configurations related to an input and an output among the functions in the system according to the first or second embodiment, and the server 101 may perform configurations that are not performed by the terminals.

For example, the terminal devices 102A and 102B may perform the configurations related to the input (for example, a configuration related to obtainment of information such as the information related to the foam, the target physical property information, the environmental condition information, and the recipe initial information), a configuration related to the output (for example, display of information for supporting the input of the information by the user at the time of obtainment of the above-described information, display of the recipe information (ratio of NCO to OH and amount of water) corresponding to the target physical property information (density and compressive strength) in which the physical property error is reduced, display of the physical property error, and the like), and processing related to information processing in the system according to the first or second embodiment.

For example, the server may perform processing of calculating the physical property information by substituting the regression formula input information into the regression formula and calculating the regression formula, and processing of calculating the recipe information (ratio of NCO to OH and amount of water) corresponding to the target physical property information (density and compressive strength) that is generated by using the generalized reduced gradient method, the regression formula, and the error function and in which the physical property error is reduced. In particular, since a calculation processing load is increased in the processing of reducing the above-described physical property error, advanced calculation processing capability such as parallel calculation processing may be used.

In addition, in the processing of calculating the physical property error at each predetermined interval within the ranges of the lower limit values and the upper limit values of the ratio of NCO to OH and the amount of water added, the calculation processing load is increased depending on the range and the interval. Since the calculation processing load is increased depending on the amount of information related to the foam in the calculation of the compressive strength per density in the regression formula and the like, advanced calculation processing capability such as parallel calculation processing may be used.

Although the embodiment in the server format has been described above, the embodiment in a cloud format may be used in addition to or instead of the server. In the cloud format, the owner of the information processing apparatus constituting the cloud, the administrator who executes the cloud, and the administrator and/or the user of the system according to the present application may be the same or different.

3.4. Other Aspects

According to a first aspect of an invention according to the present application, a program according to the first aspect causes "a computer to function as means for obtaining initial recipe information, environmental condition information, and target physical property information, means for storing a regression formula that defines a relationship between recipe information and environmental condition information, and physical property information, and means for deriving recipe information produced by applying an error function such that a difference between the target physical property information and expected physical property information is reduced, in which the expected physical property information is obtained by applying the initial recipe information and the environmental condition information to the regression formula." Here, the term "obtain" may be a concept including processing of obtaining information input by a user using an input device, processing of obtaining information from a storage device in an information processing apparatus in which obtainment means is implemented, or processing of obtaining information from an information processing apparatus different from the information processing apparatus in which the obtainment means is implemented. The processing of obtaining the information from the storage device in the information processing apparatus in which the obtainment means is implemented may include processing of obtaining the information calculated by using the technology according to the present application, and may include, for example, processing of obtaining the ratio of NCO to OH and the amount of water added set or calculated in the configuration of displaying the physical property error.

According to the first aspect, in the computer program according to a second aspect, "each of the physical property information and the target physical property information includes a density and a compressive strength, and the regression formula includes a regression formula for the density and a regression formula for the compressive strength."

According to a computer program according to a third aspect, in the first aspect or the second aspect, the program causes "the computer to function as means for generating information indicating the difference based on second recipe information, in which the information indicating the difference is associated with the second recipe information including a ratio of NCO to OH and an amount of water added on a graph indicating a relationship between the ratio of NCO to OH and the amount of water added."

According to a computer program according to a fourth aspect, in any one aspect of the first to third aspects, "the regression formula includes a constant indicating a relationship between a density and a compressive strength corresponding to a foam type."

According to a computer program according to a fifth aspect, in any one aspect of the first to fourth aspects, the program causes "the computer to function as means for generating the regression formula by using information indicating a relationship between a density and a compressive strength."

According to a computer program according to a sixth aspect, in any one aspect of the first to fifth aspects, the program causes "the computer to function as second means for obtaining first density information on a first foam type and first compressive strength information corresponding to the first density information, and second density information on a second foam type and second compressive strength information corresponding to the second density information, and third means for obtaining a third foam type, in which the means for generating the regression formula generates the information indicating the relationship between the density and the compressive strength by use of density information and compressive strength information corresponding to the third foam type and generates the regression formula."

According to a computer program according to a seventh aspect, in any one aspect of the first to sixth aspects, "the information indicating the relationship between the density and the compressive strength is a compressive strength per density."

According to a computer program according to an eighth aspect, in any one aspect of the first to seventh aspects, "the program does not include the error function."

According to a computer program according to a ninth aspect, in any one aspect of the first to eighth aspects, "the program includes the error function."

According to a computer program according to a tenth aspect, in any one of the first to ninth aspects, "the recipe information includes a ratio of NCO to OH and an amount of water added, and the environmental condition information includes a temperature, a pressure, and a humidity."

According to a computer program according to an eleventh aspect, in any one of the first to tenth aspects, "the physical property information is physical property information of a polyurethane foam."

According to a computer program according to a twelfth aspect, in any one of the first to eleventh aspects, "the polyurethane foam is either a flexible foam or a rigid foam."

A method according to a thirteenth aspect "is executed by a computer including obtaining initial recipe information, environmental condition information, and target physical property information, storing a regression formula that defines a relationship between recipe information and environmental condition information, and physical property information, and deriving recipe information produced by applying an error function such that a difference between the target physical property information and expected physical property information is reduced, in which the expected physical property information is obtained by applying the initial recipe information and the environmental condition information to the regression formula."

An apparatus according to a fourteenth aspect includes "a unit to obtain initial recipe information, environmental condition information, and target physical property information, a unit to store a regression formula that defines a relationship between recipe information, environmental condition information, and physical property information, and a unit to derive recipe information produced by applying an error function such that a difference between the target physical property information and expected physical property information is reduced, in which the expected physical property information is obtained by applying the initial recipe information and the environmental condition information to the regression formula."

The processing and procedures described in the present specification can be realized not only by the devices and the units explicitly described in the embodiments but also by software, hardware, or a combination thereof. Furthermore, the processing and procedures described in the present specification can be implemented as a computer program and can be executed by various computers. In addition, the computer program can be recorded on a non-transitory recording medium. The recording medium may be a computer-readable recording medium.

**Claims**

1. A program for causing a computer to function as

   means for obtaining initial recipe information, environmental condition information, and target physical property information,
   means for storing a regression formula that defines a relationship between recipe information and environmental condition information, and physical property information, and
   means for deriving recipe information produced by applying an error function such that a difference between the target physical property information and expected physical property information is reduced,
   wherein the expected physical property information is obtained by applying the initial recipe information and the environmental condition information to the regression formula.

2. The program according to claim 1,

   wherein each of the physical property information and the target physical property information includes a density and a compressive strength, and
   wherein the regression formula includes a regression formula for the density and a regression formula for the compressive strength.

3. The program according to claims 1 or 2, for causing the computer to function as

   means for generating information indicating the difference based on second recipe information,
   wherein the information indicating the difference is associated with the second recipe information including a ratio of NCO to OH and an amount of water added on a graph indicating a relationship between the ratio of NCO to OH and the amount of water added.

4. The program according to any one of claims 1 to 3,
   wherein the regression formula includes a constant indicating a relationship between a density and a compressive strength corresponding to a foam type.

5. The program according to any one of claims 1 to 4, for causing the computer to function as
   means for generating the regression formula by using information indicating a relationship between a density and a compressive strength.

6. The program according to claim 5, for causing the computer to function as

   second means for obtaining

   first density information on a first foam type and first compressive strength information corresponding to the first density information, and
   second density information on a second foam type and second compressive strength information corresponding to the second density information, and

   third means for obtaining a third foam type,
   wherein the means for generating the regression formula generates the information indicating the relationship between the density and the compressive strength by use of density information and compressive strength information corresponding to the third foam type and generates the regression formula.

7. The program according to claim 6,
   wherein the information indicating the relationship between the density and the compressive strength is a compressive strength per density.

8. The program according to any one of claims 1 to 7,
   wherein the program does not include the error function.

9. The program according to any one of claims 1 to 7,
   wherein the program includes the error function.

10. The program according to any one of claims 1 to 9,

    wherein the recipe information includes a ratio of NCO to OH and an amount of water added, and
    wherein the environmental condition information includes a temperature, a pressure, and a humidity.

11. The program according to any one of claims 1 to 10,
    wherein the physical property information is physical property information of a polyurethane foam.

12. The program according to claim 11,
    wherein the polyurethane foam is either a flexible foam or a rigid foam.

13. A method executed by a computer comprising:

obtaining initial recipe information, environmental condition information, and target physical property information,
storing a regression formula that defines a relationship between recipe information and environmental condition information, and physical property information, and
deriving recipe information produced by applying an error function such that a difference between the target physical property information and expected physical property information is reduced,
wherein the expected physical property information is obtained by applying the initial recipe information and the environmental condition information to the regression formula.

14. An apparatus comprising:

a unit to obtain initial recipe information, environmental condition information, and target physical property information,
a unit to store a regression formula that defines a relationship between recipe information and environmental condition information, and physical property information, and
a unit to derive recipe information produced by applying an error function such that a difference between the target physical property information and expected physical property information is reduced,
wherein the expected physical property information is obtained by applying the initial recipe information and the environmental condition information to the regression formula.

Figure 1

10

13

INPUT DEVICE
(EXAMPLE:
KEYBOARD,
MOUSE, TOUCH
PANEL)

14

DISPLAY DEVICE
(EXAMPLE:
DISPLAY)

11

16

COMPUTING
DEVICE
(EXAMPLE: CPU)

12

STORAGE DEVICE
(EXAMPLE:
MEMORY)

15

COMMUNICATION
IF (EXAMPLE:
ETHERNET)

19

Figure 2

```
┌──────────────┐    ┌─────────────────────────────────┐
│ COLLECT PAST │    │ CONSTRUCT REGRESSION FORMULA FOR│
│ MANUFACTURING│───▶│ EXPECTING FOAM PHYSICAL PROPERTY│
│ DATA         │    │ FROM RECIPE AND MANUFACTURING   │
│              │    │ ENVIRONMENT                     │
└──────────────┘    └─────────────────────────────────┘
```

```
┌─────────────────────────────────────────────────────────────────────────┐
│  ┌──────────────────┐                      ┌──────────────┐               │
│  │ ENVIRONMENTAL    │           ┌──────────│FOAM PHYSICAL │               │
│  │ CONDITION        │           │          │PROPERTY      │               │
│  └──────────────────┘     ┌───────────┐    │EXPECTED      │──┐            │
│  ┌──────────────────┐     │REGRESSION │───▶│VALUE         │  │ ┌─────────┐│
│  │ RECIPE INITIAL   │────▶│FORMULA    │    └──────────────┘  ├▶│ERROR    ││
│  │ VALUE            │     └───────────┘    ┌──────────────┐  │ │FUNCTION L││
│  └──────────────────┘                      │FOAM PHYSICAL │  │ └─────────┘│
│           ┆                                │PROPERTY      │──┘      ┆      │
│           ▼                                │TARGET VALUE  │         ▼      │
│  RECIPE OPTIMUM VALUE                      └──────────────┘  MINIMIZE (→ 0)│
└─────────────────────────────────────────────────────────────────────────┘
```

Figure 3

```
                          ┌──────────────────────────────────────┐
                          │ CALCULATE AVERAGE VALUE OF RESIN      │
                          │ STRENGTHS FOR EACH FOAM TYPE          │
                          └──────────────────────────────────────┘
                                         │
┌──────────────────┐      ┌──────────────────────────────────────┐
│ COLLECT PAST     │      │ CONSTRUCT REGRESSION FORMULA FOR      │
│ MANUFACTURING    │────▶ │ EXPECTING FOAM PHYSICAL PROPERTY      │
│ DATA             │      │ FROM RECIPE, RESIN STRENGTH, AND      │
│                  │      │ MANUFACTURING ENVIRONMENT             │
└──────────────────┘      └──────────────────────────────────────┘
                                         │
```

┌───────────────────────────────────────────────────────────────────────┐
│  ┌─────────────────────┐                ┌──────────────────┐            │
│  │ ENVIRONMENTAL       │                │ FOAM PHYSICAL    │            │
│  │ CONDITION           │     ┌────────────┐ PROPERTY       │            │
│  └─────────────────────┘     │ REGRESSION │ EXPECTED        │           │
│  ┌─────────────────────┐     │ FORMULA    │ VALUE           │           │
│  │ RECIPE INITIAL      │─────└────────────┘                 │          │
│  │ VALUE               │          ┌──────────────────┐  ┌──────────┐   │
│  └─────────────────────┘          │ FOAM PHYSICAL    │  │ ERROR    │   │
│                                   │ PROPERTY         │──│ FUNCTION L│   │
│  RECIPE OPTIMUM VALUE             │ TARGET VALUE     │  └──────────┘   │
│                                   └──────────────────┘  MINIMIZE (→ 0) │
└───────────────────────────────────────────────────────────────────────┘

Figure 4

```
        FOAM TYPE                                          ┌──────┐
                                                           └──────┘

                                     FOAM DENSITY          ┌──────┐
TARGET PHYSICAL PROPERTY                                   ├──────┤
INFORMATION                          FOAM COMPRESSIVE      └──────┘
                                     STRENGTH

                                     TEMPERATURE           ┌──────┐
ENVIRONMENTAL CONDITION                                    ├──────┤
INFORMATION                          HUMIDITY              └──────┘

RECIPE INITIAL                       RATIO OF NCO TO OH    ┌──────┐
INFORMATION                                                ├──────┤
                                     AMOUNT OF WATER ADDED  └──────┘
```

Figure 5

501

AMOUNT OF WATER

RATIO OF
NCO TO OH

502

503

Figure 6

601

SYSTEM OF PRESENT EMBODIMENT STORES
REGRESSION FORMULA CORRESPONDING TO ONE OR
PLURALITY OF FOAM TYPES.

602

SYSTEM OF PRESENT EMBODIMENT OBTAINS AND
STORES FOAM TYPE, TARGET PHYSICAL PROPERTY
INFORMATION, ENVIRONMENTAL CONDITION
INFORMATION, AND RECIPE INITIAL VALUE.

603

SYSTEM OF PRESENT EMBODIMENT SUBSTITUTES
ENVIRONMENTAL CONDITION INFORMATION AND
RECIPE INITIAL VALUE INTO REGRESSION FORMULA
CORRESPONDING TO FOAM TYPE, AND GENERATES
PHYSICAL PROPERTY INFORMATION CORRESPONDING
TO RECIPE INITIAL VALUE.

604

SYSTEM OF PRESENT EMBODIMENT DERIVES RECIPE
INFORMATION CORRESPONDING TO TARGET PHYSICAL
PROPERTY INFORMATION BY USING TARGET PHYSICAL
PROPERTY INFORMATION, ERROR FUNCTION BASED ON
PHYSICAL PROPERTY INFORMATION CORRESPONDING
TO RECIPE INITIAL VALUE, REGRESSION FORMULA,
AND GENERALIZED REDUCED GRADIENT METHOD.

605

SYSTEM OF PRESENT EMBODIMENT DISPLAYS RECIPE
INFORMATION.

Figure 7

Figure 8

801

SYSTEM OF PRESENT EMBODIMENT OBTAINS AND
STORES INFORMATION ON COMPRESSIVE STRENGTH
AND DENSITY FOR FOAM.

802

SYSTEM OF PRESENT EMBODIMENT OBTAINS AND
STORES FOAM TYPE, TARGET PHYSICAL PROPERTY
INFORMATION, ENVIRONMENTAL CONDITION
INFORMATION, AND RECIPE INITIAL VALUE.

803

SYSTEM OF PRESENT EMBODIMENT SUBSTITUTES
COMPRESSIVE STRENGTH PER DENSITY,
ENVIRONMENTAL CONDITION INFORMATION, AND
RECIPE INITIAL VALUE CORRESPONDING TO FOAM
TYPE INTO REGRESSION FORMULA, AND GENERATES
PHYSICAL PROPERTY INFORMATION CORRESPONDING
TO RECIPE INITIAL VALUE.

804

SYSTEM OF PRESENT EMBODIMENT DERIVES RECIPE
INFORMATION CORRESPONDING TO TARGET PHYSICAL
PROPERTY INFORMATION BY USING ERROR FUNCTION
BASED ON ABOVE-DESCRIBED PHYSICAL PROPERTY
INFORMATION, REGRESSION FORMULA, AND
GENERALIZED REDUCED GRADIENT METHOD.

805

SYSTEM OF PRESENT EMBODIMENT DISPLAYS RECIPE
INFORMATION

Figure 9

| FOAM TYPE | COMPRESSIVE STRENGTH 1 | COMPRESSIVE DENSITY 1 | COMPRESSIVE STRENGTH 2 | COMPRESSIVE DENSITY 2 | COMPRESSIVE STRENGTH 3 | COMPRESSIVE DENSITY 3 | COMPRESSIVE STRENGTH 4 | COMPRESSIVE DENSITY 4 | · · · |
|---|---|---|---|---|---|---|---|---|---|
| ID1 | | | | | | | | | |
| ID2 | | | | | | | | | |
| ID3 | | | | | | | | | |
| · · · | | | | | | | | | |

Figure 10

101

SERVER

103

102B

TERMINAL
DEVICE

102A

TERMINAL
DEVICE

Europäisches
Patentamt

European
Patent Office

Office européen
des brevets

## EUROPEAN SEARCH REPORT

**Application Number**

**EP 21 20 1106**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2021/023566 A1 (COVESTRO INTELLECTUAL PROPERTY GMBH & CO KG [DE]) 11 February 2021 (2021-02-11) * the whole document * ----- | 1-14 | INV. G16C60/00 |
| A | US 2021/129106 A1 (TABBI GIUSEPPE [DE] ET AL) 6 May 2021 (2021-05-06) * the whole document * ----- | 1-14 | |
| A | US 8 655 633 B2 (IBAY AUGUSTO C [US]) 18 February 2014 (2014-02-18) * the whole document * ----- | 1-14 | |
| A,D | EP 1 158 450 A2 (ROHM & HAAS [US]) 28 November 2001 (2001-11-28) * the whole document * ----- | 1-14 | |

**TECHNICAL FIELDS SEARCHED (IPC)**

G16C

The present search report has been drawn up for all claims

| Place of search | Date of completion of the search | Examiner |
|---|---|---|
| The Hague | 16 March 2022 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 1106

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2022

| Patent document cited in search report | | Publication date | Patent family member(s) | | Publication date |
|---|---|---|---|---|---|
| WO 2021023566 | A1 | 11-02-2021 | CN 114144734 A | | 04-03-2022 |
| | | | WO 2021023566 A1 | | 11-02-2021 |
| US 2021129106 | A1 | 06-05-2021 | CN 112423876 A | | 26-02-2021 |
| | | | EP 3584007 A1 | | 25-12-2019 |
| | | | EP 3806993 A1 | | 21-04-2021 |
| | | | JP 2021528533 A | | 21-10-2021 |
| | | | KR 20210019018 A | | 19-02-2021 |
| | | | US 2021129106 A1 | | 06-05-2021 |
| | | | WO 2019243115 A1 | | 26-12-2019 |
| US 8655633 | B2 | 18-02-2014 | NONE | | |
| EP 1158450 | A2 | 28-11-2001 | AU 4620401 A | | 29-11-2001 |
| | | | BR 0102105 A | | 26-12-2001 |
| | | | CA 2348070 A1 | | 24-11-2001 |
| | | | CN 1327192 A | | 19-12-2001 |
| | | | EP 1158450 A2 | | 28-11-2001 |
| | | | HK 1042358 A1 | | 09-08-2002 |
| | | | ID 30372 A | | 29-11-2001 |
| | | | JP 2002088278 A | | 27-03-2002 |
| | | | KR 20010107721 A | | 07-12-2001 |
| | | | MX PA01005203 A | | 11-08-2004 |
| | | | NZ 511867 A | | 31-05-2002 |
| | | | PL 347711 A1 | | 03-12-2001 |
| | | | TW 548571 B | | 21-08-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1158450 A

- WO 2019243115 A